# FASCICULE DE BREVET EUROPEEN

(11) **EP 0 909 427 B1**
(45) Date de publication et mention de la délivrance du brevet: **08.03.2000**
(21) Numéro de dépôt: 97930604.0
(22) Date de dépôt: 26.06.1997
(51) Int. Cl.: G06M 1/04

(54) **COMPTEUR DE DOSES**
DOSENZÄHLER
DOSIMETER

(30) Priorité: 05.07.1996 FR 9608381
(43) Date de publication de la demande: 21.04.1999
(73) Titulaire: VALOIS S.A., 27110 Le Neubourg (FR)
(72) Inventeur: BRUNA, Pascal, F-76000 Rouen (FR)
(74) Mandataire: CAPRI SARL
(86) Numéro de dépôt international: FR9701151
(87) Numéro de publication internationale: WO9801822

(56) Documents cités:
- EP-A- 0 684 047
- WO-A-95/34874
- WO-A-96/16687

## Description

La présente invention concerne un dispositif de comptage des doses de produit émises par un distributeur de produits fluides ou pulvérulents. Plus particulièrement, la présente invention concerne un dispositif compteur de doses qui permette un ou plusieurs coups à vide non comptés.

Il est connu du document WO 95/34874 un dispositif de comptage de doses qui fonctionne de manière très satisfaisante.

Toutefois, dans le cas de valves doseuses, mais aussi avec certaines pompes, il peut être souhaitable de pouvoir actionner une ou plusieurs fois le dispositif avant la première utilisation effective. Or, le dispositif divulgué dans le document WO 95/34874 compte une dose à chaque actionnement du distributeur. Il n'est donc pas possible de réaliser un ou plusieurs coups à vide, par exemple pour amorcer la pompe ou pour faire des essais avant utilisation ou même pour garantir une distribution d'une dose complète lors du premier actionnement.

La présente invention a donc pour but de réaliser un système de comptage de doses de produits émis par un distributeur de produits fluides ou pulvérulents qui ne présente pas l'inconvénient précité.

La présente invention a donc pour but de fournir un tel dispositif de comptage de doses qui ne compte des doses émises qu'après un nombre prédéterminé d'actionnement du distributeur.

La présente invention a aussi pour but de fournir un tel dispositif de comptage de doses qui soit simple et peu coûteux à fabriquer et à monter.

La présente invention a encore pour but de fournir un tel dispositif de comptage de doses qui permette de compter les doses émises par une valve doseuse.

La présente invention a également pour but de fournir un tel dispositif de comptage qui s'adapte de manière simple et peu coûteuse à des distributeurs différents, particulièrement à des distributeurs présentant différentes courses de l'organe d'actionnement.

La présente invention a donc pour objet un dispositif de comptage des doses de produit émises par un distributeur de produit fluide ou pulvérulent, ledit dispositif de comptage comportant :
- une première couronne de comptage montée rotative autour d'un axe de rotation, et
- des moyens d'entraînement pouvant coopérer avec ladite première couronne de comptage pour l'entraîner en rotation autour dudit axe, lesdits moyens d'entraînement étant actionnés à chaque actionnement du distributeur,
ledit dispositif comportant des moyens de solidarisation adaptés à solidariser lesdits moyens d'entraînement avec ladite première couronne de comptage après un nombre prédéterminé d'actionnements du distributeur, ladite première couronne de comptage n'étant entraînée en rotation qu'à partir du moment où elle est solidarisée avec lesdits moyens d'entraînement, de sorte que le dispositif de comptage ne compte les doses de produit émises qu'après ledit nombre prédéterminé d'actionnements du distributeur.

De préférence, lesdits moyens d'entraînement comporte une roue d'entraînement montée rotative autour de l'axe de rotation de ladite première couronne de comptage, à proximité de celle-ci, ladite roue d'entraînement étant entraînée en rotation autour dudit axe de rotation à chaque actionnement du distributeur.

Avantageusement, lesdits moyens de solidarisation comportent une patte solidaire de ladite roue d'entraînement, ladite patte étant sensiblement parallèle audit axe de rotation et coopérant avec une fente ménagée dans ladite première couronne de comptage, ladite patte venant en butée contre une paroi d'extrémité de ladite fente après ledit nombre prédéterminé d'actionnement du distributeur.

Avantageusement, ladite fente s'étend en arc de cercle dans ladite première couronne de comptage, chaque actionnement du distributeur entraînant la roue d'entraînement en rotation autour dudit axe de rotation, ladite patte se déplaçant simultanément dans ladite fente sans entraîner la première couronne de comptage en rotation, jusqu'à ce que ladite patte vienne en butée contre la paroi radiale d'extrémité de la fente, tout actionnement ultérieur du distributeur amenant la patte de la roue d'entraînement à entraîner ladite première couronne de comptage en rotation autour dudit axe de rotation pour compter les doses de produit émises, la longueur de la fente définissant ainsi ledit nombre prédéterminé d'actionnements du distributeur pendant lequel la couronne de comptage ne tourne pas.

En particulier, ladite première couronne de comptage et ladite roue d'entraînement comportent chacune un dispositif anti-retour respectif, empêchant une rotation en sens inverse de ladite première couronne et de ladite roue d'entraînement.

De préférence, le distributeur comporte un organe d'actionnement déplaçable entre une position de repos et une position d'actionnement, de telle sorte qu'à chaque actionnement du distributeur, ledit organe d'actionnement coopère avec ladite roue d'entraînement pour la faire tourner d'un angle prédéterminé autour dudit axe de rotation.

Avantageusement, ledit organe d'actionnement se déplace dans une direction parallèle à l'axe de rotation de ladite roue d'entraînement et de ladite première couronne de comptage.

Avantageusement, ladite roue d'entraînement comporte deux séries d'indentations disposées circonférentiellement et saillantes radialement vers l'extérieur, les indentations de la première série d'indentations présentant une première surface oblique qui coopère avec l'organe d'actionnement lorsqu'il se déplace de sa position de repos vers sa position d'actionnement, et les indentations de la seconde série d'indentations présentant une seconde surface oblique qui coopère avec l'organe d'actionnement lorsqu'il se déplace de sa position d'actionnement vers sa position de repos, de sorte que ladite roue d'entraînement tourne dudit angle prédéterminé pendant un cycle d'actionnement de l'organe d'actionnement.

En particulier, ledit distributeur comporte une valve doseuse actionnée par un poussoir, ledit poussoir incorporant ledit organe d'actionnement de sorte que lorsque l'utilisateur appuie sur le poussoir pour délivrer une dose, la roue d'entraînement tourne sur une partie dudit angle prédéterminé, et lorsque le poussoir est ramené vers sa position initiale, ladite roue d'entraînement tourne du restant dudit angle prédéterminé.

Eventuellement, le dispositif comporte en outre une seconde couronne de comptage montée rotative autour dudit axe de rotation et actionnée en rotation par ladite première couronne de comptage chaque fois que ladite première couronne de comptage a effectué une rotation de 360° autour dudit axe de rotation. Dans ce cas, la coopération entre la première couronne de comptage et la seconde couronne de comptage peut être similaire à celle décrite dans le document WO 95/34874.

D'autres caractéristiques et avantages de la présente invention apparaîtront au cours de la description détaillée suivante, donnée à titre d'exemple non limitatif, en regard des dessins joints, sur lesquels :
- la figure 1 est une une vue schématique éclatée d'un dispositif de comptage selon la présente invention,
- la figure 2 est une vue schématique en coupe verticale du dispositif de comptage de l'invention,
- la figure 3 est une vue schématique de la roue d'entraînement selon l'invention,
- la figure 4 est une vue schématique de ladite roue d'entraînement montrant la coopération entre l'organe d'entraînement et ladite roue d'entraînement pendant un cycle d'actionnement du distributeur,
- la figure 5 est une vue schématique en perspective de la première couronne de comptage selon la présente invention, et
- la figure 6 est une vue schématique en coupe horizontale montrant la coopération entre la première couronne de comptage et la seconde couronne de comptage.

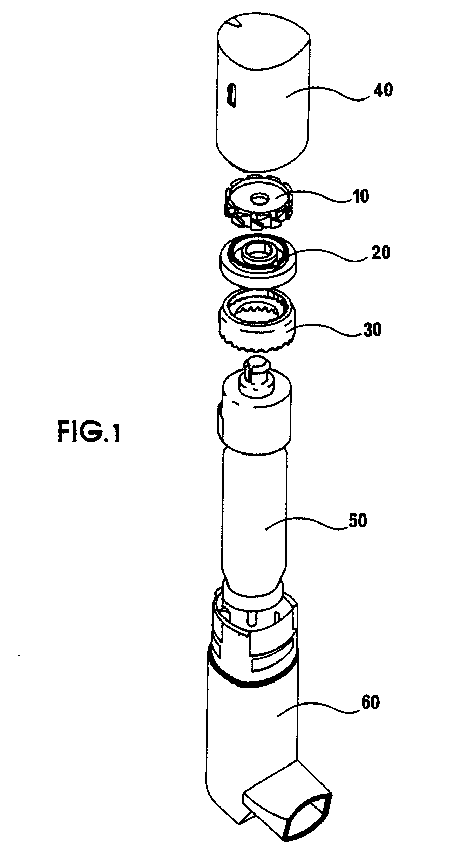

En référence aux figures 1, 2, 5 et 6, le dispositif de comptage des doses de produit émises par un distributeur de produit fluide ou pulvérulent de l'invention est du type de celui du document WO 95/34874. Le fonctionnement de ce dispositif ne sera donc ci-après décrit que brièvement et le document précité est incorporé ici à titre de référence pour tout ce qui concerne le fonctionnement général du dispositif de comptage et en particulier ce qui concerne le comptage effectif des doses de produit émises.

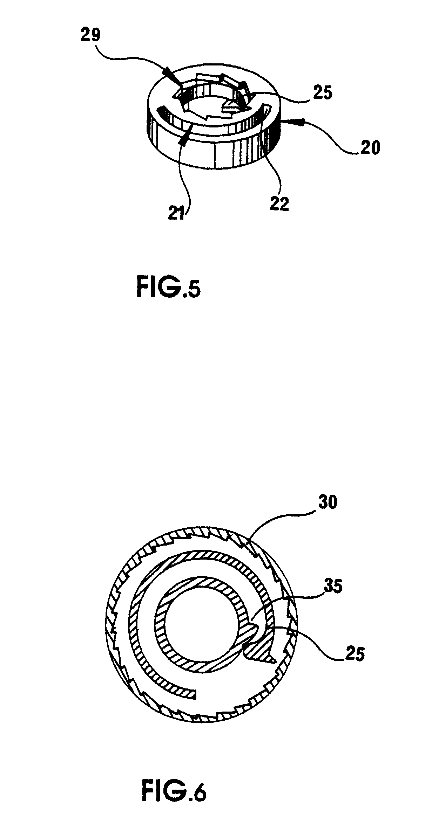

Comme visible sur les figures 1, 2, 5 et 6, le dispositif de comptage comprend au moins une première couronne de comptage 20 et une ou plusieurs autres couronnes de comptage. En particulier, le présent dispositif comporte une seconde couronne de comptage 30. La première couronne de comptage 20 est entraînée en rotation autour de son axe de rotation 1 par l'actionnement du distributeur. Comme visible sur la figure 6, la seconde couronne de comptage 30 est entraînée en rotation par ladite première couronne de comptage 20 lorsque cette dernière a fait un tour complet autour de son axe 1. Ainsi les première et seconde couronnes de comptage 20, 30 sont particulièrement adaptées à compter respectivement les unités et les dizaines. La première couronne de comptage 20 comporte avantageusement une patte flexible 25 qui peut coopérer avec une came fixe 35 à chaque fois qu'elle a fait un tour complet autour de l'axe de rotation 1. Lorsque la patte 25 coopère avec la came 35, elle est forcée en prise avec ladite seconde couronne de comptage 30 pour entraîner celle-ci également en rotation autour dudit axe 1. La première et la seconde couronne de comptage peuvent bien entendu comporter des systèmes anti-retour pour éviter que ces deux couronnes de comptage ne tournent dans le sens inverse à celui qui leur est imposé par l'actionnement du distributeur. En particulier, la première couronne de comptage 20 peut comporter une denture interne 29 qui coopère avec un cliquet fixe par rapport l'axe de rotation 1. Le principe du dispositif de comptage est donc le même que celui divulgué dans le document WO 95/34874.

Toutefois, dans ce dispositif, la première couronne de comptage 20 est entraînée en rotation à chaque actionnement du distributeur. Il est donc impossible dans le dispositif de l'art antérieur de réaliser des coups à vide, c'est-à-dire non comptés, du distributeur avant de commencer à compter la première dose.

La présente invention concerne donc un dispositif de comptage qui permette justement de pouvoir actionner le distributeur un nombre prédéterminé de fois avant que le dispositif de comptage ne commence de compter les doses émises. Alors que dans les dispositifs de l'art antérieur, la première couronne de comptage est entraînée en rotation à chaque actionnement du distributeur, la présente invention prévoit que la première couronne de comptage 20 ne sera entraînée en rotation, et donc ne comptera les doses émises, qu'à partir d'un nombre prédéterminé d'actionnement dudit distributeur. Pour ce faire, l'invention prévoit d'entraîner la première couronne de comptage 20 au moyen d'un moyen d'entraînement 10, avantageusement une roue d'entraînement, qui est montée rotative autour du même axe de rotation 1.

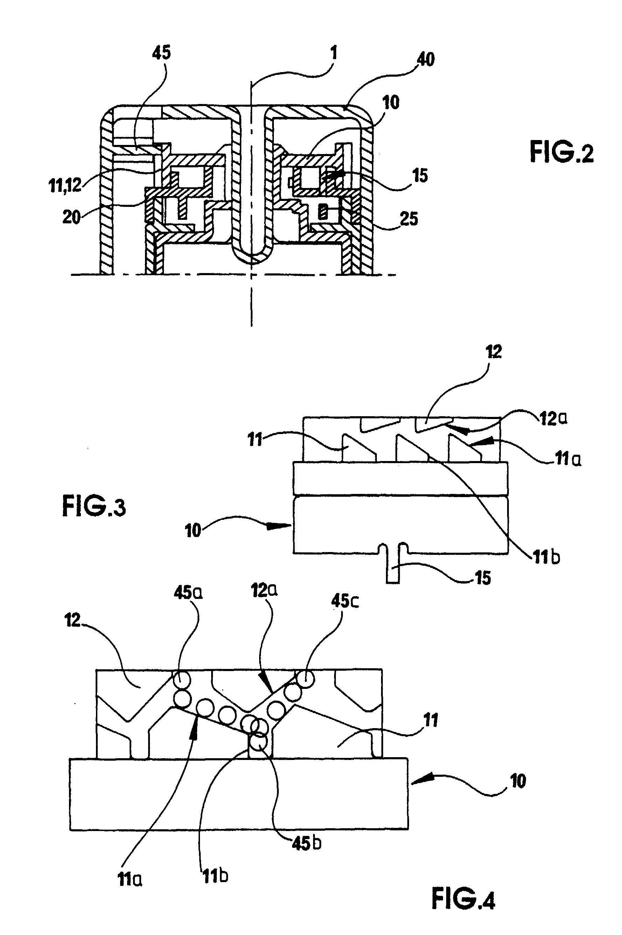

Cette roue d'entraînement 10 est entraînée en rotation autour de l'axe 1 à chaque actionnement du distributeur mais elle ne coopère avec la première couronne de comptage 20 pour entraîner cette dernière en rotation autour de l'axe 1 qu'à partir dudit nombre prédéterminé d'actionnement du distributeur. Ce dispositif comporte donc des moyens de solidarisation adaptés à solidariser la roue d'entraînement 10 avec la première couronne de comptage 20 après le nombre prédéterminé d'actionnement du distributeur. Ces moyens de solidarisation comprennent avantageusement une patte 15 fixée à ou faisant partie intégrante de la roue d'entraînement 10 et qui coopère avec une fente correspondante 21 ménagée dans ladite première couronne de comptage 20. En référence aux figures 2 et 3, on voit que la patte 15 est solidaire de ladite roue d'entraînement 10 et s'étend sensiblement parallèlement audit axe de rotation 1. D'autre part, il apparaît sur la figure 5 que ladite fente 21 ménagée dans ladite première couronne de comptage 20 s'étend avantageusement en arc de cercle dans ladite première couronne de comptage 20. Ainsi, à chaque actionnement du distributeur, la roue d'entraînement 10 est entraînée en rotation, ladite patte 15 se déplaçant simultanément dans ladite fente 21 sans entraîner la première couronne de comptage 20 en rotation. Ce n'est que lorsque ladite patte 15 vient en butée contre la paroi radiale d'extrémité 22 de la fente 21 que, lors d'un actionnement ultérieur du distributeur, la patte 15 de la roue d'entraînement 10 entraînera la première couronne de comptage 20 en rotation autour de l'axe de rotation 1, pour commencer à compter les doses de produit émises. Par conséquent, la longueur de ladite fente 21 détermine le nombre prédéterminé d'actionnements du distributeur pendant lesquels la première couronne de comptage 20 ne tourne pas et donc pendant lequel le dispositif de comptage ne compte pas les doses de produit émises.

Evidemment, la roue d'entraînement 10 peut également comporter un dispositif anti-retour similaire à celui de la figure 5 pour la première couronne de comptage 20, à savoir une denture interne coopérant avec un système de cliquets fixe par rapport à l'axe de rotation 1. Ainsi, la roue d'entraînement 10 et la première couronne de comptage 20 ne peuvent tourner que dans le sens imposé par le fonctionnement du dispositif de comptage.

La roue d'entraînement 10 est elle entraînée en rotation autour de l'axe 1 à chaque actionnement du distributeur. Cette roue d'entraînement 10 s'applique donc à tous les dispositifs de comptage tels que divulgués dans le document WO 95/34874 et plus généralement à tous les dispositifs de comptage actionnés par l'actionnement du distributeur.

En référence aux figures 1 à 4, il va maintenant être décrit un mode de réalisation particulier de ladite roue d'entraînement 10, particulièrement adapté à un dispositif de comptage fonctionnant dans un distributeur actionné par un organe d'actionnement se déplaçant dans une direction parallèle à l'axe de rotation 1 de la roue d'entraînement 10 et de la première couronne de comptage 20.

Plus particulièrement, le distributeur peut comporter une valve doseuse 50 actionnée par un poussoir 40, ledit poussoir 40 incorporant un organe d'actionnement 45 sous la forme d'une patte s'étendant radialement par rapport à l'axe de rotation 1 du dispositif de comptage, comme visible sur la figure 2. La valve doseuse comporte en outre un embout buccal 60 par lequel la dose de produit est expulsée en direction de l'utilisateur. Il est clair, qu'à la place d'une valve doseuse 50 dans le dispositif représenté sur la figure 1, on pourrait prévoir tout autre type d'organe de distribution, tel qu'une pompe.

En référence aux figures 1 et 2, le poussoir 40 est donc déplaçable entre une position de repos et une position d'actionnement. Ce poussoir 40 incorpore un organe d'actionnement 45 qui est donc également déplaçable entre une position d'actionnement et une position de repos. Cet organe d'actionnement 45 est réalisé de telle sorte qu'à chaque actionnement de la valve ou du dispositif en général, il coopère avec la roue d'entraînement 10 pour la faire tourner d'un angle déterminé autour de l'axe de rotation 1. Pour ce faire, la roue d'entraînement 10 comporte avantageusement deux séries d'indentations 11 et 12 disposées circonférentiellement, tel que représenté sur les figures 3 et 4. Ces indentations sont de préférence saillantes vers l'extérieur pour pouvoir coopérer avec ledit organe d'actionnement 45. Avantageusement, les indentations 11 de la première série d'indentations présentent une première surface oblique 11a qui coopère avec l'organe d'actionnement 45 lorsqu'il se déplace de sa position de repos vers de sa position d'actionnement, et les indentations 12 de la seconde série d'indentations présentent une seconde surface oblique 12a qui coopère avec l'organe d'actionnement 45 lorsqu'il retourne de sa position d'actionnement vers sa position de repos. Par conséquent, ladite roue d'entraînement 10 tourne dudit angle déterminé pendant un cycle complet d'actionnement de l'organe d'actionnement 45. Plus particulièrement, comme visible sur la figure 3, la première surface oblique 11a de la première série d'indentations est circonférentiellement décalée par rapport à la seconde surface oblique 12a de la seconde série d'indentations 12. La figure 4 représente schématiquement des positions successives de l'organe d'actionnement 45 pendant un cycle d'actionnement du poussoir 40. Ainsi, lorsque le poussoir est encore dans sa position de repos, l'organe d'actionnement adopte la position 45a. L'utilisateur appuie alors sur le poussoir 40 et l'organe d'actionnement 45 va descendre jusqu'à ce qu'il rencontre la première surface oblique 11a d'une première indentation 11. A partir de ce moment-là, une continuation du déplacement du poussoir 40 va créer sur ladite indentation 11 une force de pression comportant une composante non verticale de sorte que la roue d'entraînement 10 est entraînée en rotation. Lorsque le poussoir 40 arrive en position d'actionnement, l'organe d'actionnement 45 adopte la position référencée par 45b. A ce moment-là, la roue d'entraînement 10 a effectué une partie seulement de l'angle de rotation déterminé sur lequel elle tourne à chaque entraînement du distributeur. Lorsque l'utilisateur relâche le poussoir 40 après que la dose a été émise, ce poussoir 40 va être ramené vers sa position de repos, par exemple par un ressort, et l'organe d'actionnement 45 va donc remonter verticalement jusqu'à ce qu'il rencontre la seconde surface oblique 12a d'une seconde indentation 12. A ce moment-là, l'organe d'actionnement 45 exerce sur cette indentation 12 une force de compression comportant également une composante non verticale de sorte que la roue d'entraînement est à nouveau entraînée en rotation autour de l'axe de rotation. Les surfaces obliques des premières séries d'indentations 12 et des secondes séries d'indentations 12 sont orientées de telle manière que les deux composantes verticales des forces de compression qui s'exercent respectivement sur les deux séries d'indentations font tourner la roue d'entraînement dans le même sens de rotation. Ainsi, lorsque le poussoir 40 revient à sa position de repos après un cycle d'actionnement complet du distributeur, l'organe d'actionnement 45 adopte la position référencée 45c et la roue d'entraînement a effectué une rotation de l'angle déterminé autour de l'axe de rotation. Bien entendu, si les moyens de solidarisation, c'est-à-dire la patte 15 et la fente 21 coopèrent, la roue d'entraînement 10 entraîne simultanément en rotation la première couronne de comptage 20 et une dose émise est comptée par le dispositif de comptage. Il est clair que dans ce cas, l'angle de rotation de la première couronne de comptage 20 lors d'un actionnement du distributeur est le même que celui de la roue d'entraînement 10.

Il est particulièrement avantageux, comme représenté sur les figures 3 et 4 que la première surface oblique 11a de la première série d'indentations 11 se prolonge par une section verticale 11b. Lorsque l'organe d'actionnement 45 se déplace dans cette section, il n'exerce plus sur la première série d'indentations 11 de force de compression avec une composante non verticale. Cette section verticale 11b est destinée à permettre d'amener le poussoir 40 jusqu'à sa position d'actionnement. Ainsi, la roue d'entraînement 10 de la présente invention est adaptée à toutes les valves doseuses existantes quelle que soit leur course d'actionnement du poussoir. Si la valve doseuse comporte une course d'actionnement petite, alors l'organe d'actionnement ne parviendra pas au fond de la section 11b lors de l'actionnement du poussoir et passera directement de la première surface oblique 11a sur la seconde surface oblique 12a. Par contre, si la valve doseuse a une course d'actionnement plus grande, alors la section 11b permet de compenser cette différence de course et n'oblige pas de modifier la roue d'entraînement pour chaque valve doseuse différente. Ceci est également vrai pour les pompes qui, selon le volume de la dose émise, comportent également des courses d'actionnement différentes. Ainsi, le dispositif de l'invention permet de s'adapter à tout type de pompe ou de valves-doseuses existant. Il s'ensuit une économie de coûts importante et une simplicité de fabrication et de montage.

Le nombre prédéterminé d'actionnement du distributeur pendant lequel le dispositif de comptage ne compte pas, c'est-à-dire la longueur de la fente 21 ménagée dans la première couronne de comptage 20, est déterminé à l'avance en tenant compte d'un certain nombre d'actionnements que l'on souhaite effectuer avant que l'utilisateur du produit n'émette de manière effective la première dose pour se traiter. Ces coups à vide peuvent par exemple comprendre un ou plusieurs coups

Bien que la présente invention ait été décrite en relation avec un dispositif de comptage similaire à celui divulgué dans le document WO 95/34874, il est clair qu'il s'adapte à tout type de dispositif de comptage qui est destiné à compter une dose de produit lors de chaque actionnement d'un distributeur. De même, bien que la présente invention ait été particulièrement décrite en relation à un distributeur où l'organe d'actionnement exerce une course parallèle à l'axe de rotation du dispositif de comptage, comme c'est le cas pour une valve-doseuse par exemple, il est entendu que l'invention s'applique à tout type de distributeur, quel que soit son actionnement.

## Revendications

1. Dispositif de comptage des doses de produit émises par un distributeur de produit fluide ou pulvérulent, ledit dispositif de comptage comportant :
- une première couronne de comptage (20) montée rotative autour d'un axe de rotation (1), et
- des moyens d'entraînement (10) pouvant coopérer avec ladite première couronne de comptage (20) pour l'entraîner en rotation autour dudit axe (1), lesdits moyens d'entraînement (10) étant actionnés à chaque actionnement du distributeur,
caractérisé en ce que ledit dispositif comporte des moyens de solidarisation (15, 22) adaptés à solidariser lesdits moyens d'entraînement (10) avec ladite première couronne de comptage (20) après un nombre prédéterminé d'actionnements du distributeur, ladite première couronne de comptage (20) n'étant entraînée en rotation qu'à partir du moment où elle est solidarisée avec lesdits moyens d'entraînement (10), de sorte que le dispositif de comptage ne compte les doses de produit émises qu'après ledit nombre prédéterminé d'actionnements du distributeur.

2. Dispositif selon la revendication 1, dans lequel lesdits moyens d'entraînement comporte une roue d'entraînement (10) montée rotative autour de l'axe de rotation (1) de ladite première couronne de comptage (20), à proximité de celle-ci, ladite roue d'entraînement (10) étant entraînée en rotation autour dudit axe de rotation à chaque actionnement du distributeur.

3. Dispositif selon la revendication 2, dans lequel lesdits moyens de solidarisation comportent une patte (15) solidaire de ladite roue d'entraînement (10), ladite patte (15) étant sensiblement parallèle audit axe de rotation (1) et coopérant avec une fente (21) ménagée dans ladite première couronne de comptage (20), ladite patte (15) venant en butée contre une paroi d'extrémité (22) de ladite fente (21) après ledit nombre prédéterminé d'actionnement du distributeur.

4. Dispositif selon la revendication 3, dans lequel ladite fente (21) s'étend en arc de cercle dans ladite première couronne de comptage (20), chaque actionnement du distributeur entraînant la roue d'entraînement (10) en rotation autour dudit axe de rotation (1), ladite patte (15) se déplaçant simultanément dans ladite fente (21) sans entraîner la première couronne de comptage (20) en rotation, jusqu'à ce que ladite patte (15) vienne en butée contre la paroi radiale d'extrémité (22) de la fente (21), tout actionnement ultérieur du distributeur amenant la patte (15) de la roue d'entraînement (10) à entraîner ladite première couronne de comptage (20) en rotation autour dudit axe de rotation (1) pour compter les doses de produit émises, la longueur de la fente (21) définissant ainsi ledit nombre prédéterminé d'actionnements du distributeur pendant lequel la couronne de comptage (20) ne tourne pas.

5. Dispositif selon l'une quelconque des revendications 2 à 4, dans lequel ladite première couronne de comptage et ladite roue d'entraînement comportent chacune un dispositif anti-retour respectif, empêchant une rotation en sens inverse de ladite première couronne et de ladite roue d'entraînement.

6. Dispositif selon l'une quelconque des revendications 2 à 5, dans lequel le distributeur comporte un organe d'actionnement (45) déplaçable entre une position de repos et une position d'actionnement, de telle sorte qu'à chaque actionnement du distributeur, ledit organe d'actionnement (45) coopère avec ladite roue d'entraînement (10) pour la faire tourner d'un angle prédéterminé autour dudit axe de rotation.

7. Dispositif selon la revendication 6, dans lequel ledit organe d'actionnement (45) se déplace dans une direction parallèle à l'axe de rotation (1) de ladite roue d'entraînement (10) et de ladite première couronne de comptage (20).

8. Dispositif selon la revendication 7, dans lequel ladite roue d'entraînement (10) comporte deux séries d'indentations (11, 12) disposées circonférentiellement et saillantes radialement vers l'extérieur, les indentations (11) de la première série d'indentations présentant une première surface oblique (11a) qui coopère avec l'organe d'actionnement (45) lorsqu'il se déplace de sa position de repos vers sa position d'actionnement, et les indentations (12) de la seconde série d'indentations présentant une seconde surface oblique (12a) qui coopère avec l'organe d'actionnement (45) lorsqu'il se déplace de sa position d'actionnement vers sa position de repos, de sorte que ladite roue d'entraînement (10) tourne dudit angle prédéterminé pendant un cycle d'actionnement de l'organe d'actionnement (45).

9. Dispositif selon la revendication 8, dans lequel ledit distributeur comporte une valve doseuse (50) actionnée par un poussoir (40), ledit poussoir (40) incorporant ledit organe d'actionnement (45) de sorte que lorsque l'utilisateur appuie sur le poussoir (40) pour délivrer une dose, la roue d'entraînement (10) tourne sur une partie dudit angle prédéterminé, et lorsque le poussoir (40) est ramené vers sa position initiale, ladite roue d'entraînement (10) tourne du restant dudit angle prédéterminé.

10. Dispositif selon l'une quelconque des revendications précédentes, comportant en outre une seconde couronne de comptage (30) montée rotative autour dudit axe de rotation (1) et actionnée en rotation par ladite première couronne de comptage (20) chaque fois que ladite première couronne de comptage (20) a effectué une rotation de 360° autour dudit axe de rotation (1).

## Patentansprüche

1. Vorrichtung zum Zählen der Produktdosissen, die von einer Abgabevorrichtung für ein Fluid oder Pulver abgegeben werden, wobei diese Zählvorrichtung folgende Bestandteile umfaßt:
- einen ersten Zählkranz (20), der um eine Rotationsachse (1) drehbar montiert ist, und
- Antriebseinrichtungen (10), die mit dem ersten Zählkranz (20) zusammenwirken können, um ihn für eine Drehung um die besagte Achse (1) anzutreiben, wobei die Antriebseinrichtungen (10) bei jeder Betätigung der Abgabevorrichtung betätigt werden,
dadurch **gekennzeichnet**, daß die Vorrichtung Verbindungseinrichtungen (15, 22) umfaßt, die geeignet sind, die Antriebseinrichtungen (10) mit dem ersten Zählkranz (20) nach einer vorbestimmten Anzahl von Betätigungen der Abgabevorrichtung zu verbinden, so daß der erste Zählkranz (20) erst von dem Augenblick an, in welchem er mit den besagten Antriebseinrichtungen (10) verbunden ist, für eine Drehung angetrieben wird, so daß die Zählvorrichtung die Dosissen des abgegebenen Produktes erst nach der vorbestimmten Anzahl von Betätigungen der Abgabevorrichtung zählt.

2. Vorrichtung nach Anspruch 1, bei der die Antriebseinrichtungen ein Antriebsrad (10) umfassen, das um die Drehachse (1) des ersten Zählkranzes (20) in dessen Nähe drehbar montiert ist, wobei das Antriebsrad (10) bei jeder Betätigung der Abgabevorrichtung für eine Drehung um besagte Drehachse angetrieben wird.

3. Vorrichtung nach Anspruch 2, bei der die Einrichtungen zum Verbinden eine mit dem Antriebsrad (10) fest verbundene Klaue (15) umfassen, wobei sich die Klaue (15) im wesentlichen parallel zur Drehachse (1) erstreckt und mit einem Schlitz (21) zusammenwirkt, der in dem ersten Zählkranz (20) vorgesehen ist, wobei die Klaue (15) an einer Endwand (22) des Schlitzes (21) nach der vorbestimmten Anzahl von Betätigungen der Abgabevorrichtung zur Anlage kommt.

4. Vorrichtung nach Anspruch 3, bei der der Schlitz (21) sich in einem Kreisbogen in dem ersten Zählkranz (20) erstreckt, wobei jede Betätigung der Abgabevorrichtung das Antriebsrad (10) für eine Drehung um besagte Drehachse (1) antreibt und sich die Klaue (15) gleichzeitig in dem Schlitz (21) verschiebt, ohne den ersten Zählkranz (20) für eine Drehung anzutreiben, bis diese Klaue (15) an der radialen Endwand (22) des Schlitzes (21) zur Anlage kommt, so daß jede weitere Betätigung der Abgabevorrichtung die Klaue (15) des Antriebsrades (10) dazu bringt, den ersten Zählkranz (20) für eine Drehung um die Drehachse (1) anzutreiben, um die abgegebenen Dosissen des Produktes zu zählen, so daß somit die Länge des Schlitzes (21) die vorbestimmte Anzahl von Betätigungen der Abgabevorrichtung definiert, während derer sich der Zählkranz (20) nicht dreht.

5. Vorrichtung nach einem der Ansprüche 2 bis 4, bei der der erste Zählkranz und das Antriebsrad jeweils eine entsprechende Umkehrsperre umfassen, die eine Drehung im entgegengesetzten Drehsinn des ersten Kranzes und des Antriebsrades verhindern.

6. Vorrichtung nach einem der Ansprüche 2 bis 5, bei der die Abgabevorrichtung ein Betätigungsorgan (45), das zwischen einer Ruhelage und einer Betätigungsstellung verschiebbar ist, derart umfaßt, daß bei jeder Betätigung der Abgabevorrichtung das Betätigungsorgan (45) mit dem Antriebsrad (10) zusammenwirkt, um es zu einer Drehung um einen vorbestimmten Winkel um die Drehachse zu veranlassen.

7. Vorrichtung nach Anspruch 6, bei der sich das Betätigungsorgan (45) in einer zur Drehachse (1) des Antriebsrades (10) und des ersten Zählkranzes (20) parallelen Richtung verschiebt.

8. Vorrichtung nach Anspruch 7, bei der das Antriebsrad (10) zwei Gruppen von Zacken (11, 12) umfaßt, die in Umfangsrichtung angeordnet sind und radial nach außen vorspringen, wobei die Zacken (11) der ersten Gruppe von Zacken eine erste schräge Oberfläche (11a) aufweisen, die mit dem Betätigungsorgan (45) zusammenwirkt, wenn es sich aus seiner Ruhelage zu seiner Arbeitsstellung hin verschiebt, und wobei die Zacken (12) der zweiten Gruppe von Zacken eine zweite schräge Oberfläche (12a) besitzen, die mit dem Betätigungsorgan (45) zusammenwirkt, wenn sich dieses aus seiner Betätigungsstellung zu seiner Ruhelage hinverschiebt, so daß das Antriebsrad (10) sich um den vorbestimmten Winkel während eines Betätigungszyklus des Betätigungsorgans (45) dreht.

9. Vorrichtung nach Anspruch 8, bei der die Abgabevorrichtung ein Dosierventil (50) umfaßt, das von einem Drücker (40) betätigt wird, wobei der Drücker (40) das Betätigungsorgan (45) derart umfaßt, daß dann, wenn der Verwender auf den Drücker (40) drückt, um eine Dosis abzugeben, sich das Antriebsrad (10) um einen Teil des vorbestimmten Winkels dreht und daß sich das Antriebsrad (10) dann, wenn der Drücker (40) in seine Anfangsposition zurückgebracht wird, um den Rest dieses vorbestimmten Winkels dreht.

10. Vorrichtung nach einem der vorhergehenden Ansprüche, die im übrigen einen zweiten Zählkranz (30) umfaßt, der um die besagte Drehachse (1) drehbar montiert ist und vom ersten Zählkranz (20) jedes Mal dann für eine Drehung betätigt wird, wenn der erste Zählkranz (20) eine Drehung um 360 ° um die Drehachse (1) ausgeführt hat.

## Claims

1. Apparatus for measuring doses of products dispensed by a fluid or powdery product dispenser, said apparatus for measuring comprising:
- a first metering ring (20) mounted to rotate around a rotation axis (1), and
- drive means (10) capable of co-operating with said first metering ring (20) to drive said metering ring to rotate around said axis (1), said drive means (10) being actuated each time the dispenser is actuated,
characterized by the fact that said apparatus comprises interlocking means (15, 22) that are used to engage said drive means (10) with said first metering ring (20) after the dispenser has been actuated a predetermined number of times, said first metering ring (20) only being driven to rotate once it is engaged with said drive means (10), such that the apparatus for measuring only counts the doses of dispensed product after the dispenser has been actuated said predetermined number of times.

2. Apparatus of claim 1 wherein the said drive means comprise a drive wheel (10) that is mounted to rotate around the rotation axis (1) of said first metering ring (20) near to said first metering ring (20), said drive wheel (10) being driven to rotate around said rotation axis each time the dispenser is actuated.

3. Apparatus of claim 2 wherein said interlocking means comprise a foot (15) that is an integrated part of said drive wheel (10), said foot (15) being more or less parallel to said rotation axis (1) and operating in conjunction with a slot (21) that is provided in said first metering ring (20), the said foot (15) coming to bear on an end surface (22) of said slot (21) after the dispenser has been actuated a predetermined number of times.

4. Apparatus of claim 3 wherein said slot (21) lies in the arc of a circle in said first metering ring (20), each time the dispenser is actuated driving drive wheel (10) to rotate around said rotation axis (1), said foot (15) being simultaneously displaced in said slot (21) without causing first metering ring (20) to rotate until said foot (15) comes to bear on radial surface end (22) of slot (21), any subsequent actions of the dispenser bringing foot (15) of the drive wheel (10) to drive said first metering ring (20) to rotate around said rotation axis (1) in order to count the doses of dispensed product, the length of the slot thus constituting said predetermined number of times that the dispenser is actuated during which the metering ring (20) does not turn.

5. Apparatus of any of claims 2 to 4 wherein said first metering ring and said drive wheel each include a respective anti-return device that prevents said first metering ring and said drive wheel from rotating in opposite directions.

6. Apparatus of any of claims 2 to 5 wherein the dispenser comprises an actuating device (45) that is capable of being displaced between a rest position and an actuating position such that each time the dispenser is actuated said actuating device (45) operates in conjunction with said drive wheel (10) to rotate said drive wheel through a predetermined angle around said rotation axis.

7. Apparatus of claim 6 wherein said actuating device (45) is displaced in a direction parallel to rotation axis (1) of said drive wheel (10) and of said first metering ring (20).

8. Apparatus of claim 7 wherein said drive wheel (10) comprises two rows of indentations (11,12) that are arranged around the circumference and that project radially outwards, the indentations (11) of the first row of indentations have a first oblique surface (11a) that operates in conjunction with the actuating device (45) when the device is displaced from the rest position to the actuating position and the indentations (12) of the second row have a second oblique surface (12a) that operates in conjunction with the actuating device (45) when the device is displaced from the actuating position to the rest position, such that the said drive wheel (10) rotates through said predetermined angle during an actuating cycle of the actuating device (45).

9. Apparatus of claim 8 wherein said dispenser comprises a dispensing valve (50) actuated by a push-button (40), said push-button (40) including said actuating device (45) such that when the user depresses push-button (40) to obtain a dose the drive wheel (10) rotates through a section of said predetermined angle and when push-button (40) is brought back to its initial position said drive wheel (10) rotates through the remainder of the said predetermined angle.

10. Apparatus according to any of the preceding claims also comprising a second metering ring (30) that is mounted to rotate around said rotation axis (1) and which is actuated to rotate by said first metering ring (20) each time that said first metering ring (20) completes a 360° rotation around said rotation axis (1) .
